# EUROPEAN PATENT SPECIFICATION

(11) **EP 0 601 891 B1**
(45) Date of publication and mention of the grant of the patent: **17.04.1996**
(21) Application number: 93310020.8
(22) Date of filing: 13.12.1993
(51) Int. Cl.: A61K 9/08, A61K 33/00, A61K 33/20, A61K 33/40, A61K 31/09, A61K 31/77

(54) **Antifungal and skin healing promoting agent**
Pilzhemmendes und hautheilungsförderndes Mittel
Agent antifongique et pour favoriser la cicatrisation de la peau

(30) Priority: 11.12.1992 JP 359717/92
(43) Date of publication of application: 15.06.1994
(73) Proprietor: JAPAN LOTION COMPANY, Zama City, Kanagawa Prefecture (JP)
(72) Inventor: Uehara, Kazutoyo, Zama City, Kanagawa Prefecture (JP)
(74) Representative: Matthews, Derek Peter

(56) References cited:
- DE-A- 3 403 631

## Description

### 1. BACKGROUND OF INVENTION

### 1.1 Field of the Invention

This invention relates to a therapeutic agent for the treatment of skin disease (infecticosa eczematoides or Engmaris disease) caused by trichophytons, eczema or various fungi, and also for use in activating the recovery of the skin after disease or burn injury.

More particularly, this invention relates to a therapeutic agent for the treatment of skin disease such as athlete's foot, ringworm and tinea by oxidation, reduction, bleaching and fungicidal activity of the agent, characterised in that trichophytons or various fungi in a deep layer of the skin are allured by oxygen contained in the antifungal agent to or near the surface of the skin for easy sterilisation. The agent is also capable of activating the early recovery of the skin after disease or burn injury.

### 1.2 Description of the Prior Art

When trichophytons causing athlete's foot and/or ringworm are in the epidermis of the skin, it is comparatively easy to sterilise them. When they are in a deep layer such as keratin or dermis of the skin, complete sterilisation of trichophytons cannot be expected, thus leaving the skin disease beyond medical treatment.

Some antifungal agents containing econazole nitrate, phydone derivative or miconazole nitrate for the remedy of athlete's foot and ringworm have been proposed, in which keratin or the epidermis and the dermis of the skin are corroded by another medicine.

Since the healthy skin is also corroded, the early recovery of the skin affected with dermatophytoses, eczema or various fungi cannot be expected.

Another conventional antifungal agent containing fluoride for the remedy of athlete's foot or ringworm has also been proposed, but fluoride not only sterilises trichophytons, but also corrodes the healthy epidermis, dermis and deep skin and even the bone as well.

DE-A-3403631 describes an antifungal composition comprising peroxy-compounds and chlorite.

### 2. SUMMARY OF THE INVENTION

A principal object of this invention is to provide an antifungal agent for the treatment of skin disease such as athlete's foot, ringworm and tinea by oxidation, reduction, bleaching and fungicidal activity of the antifungal agent which is characterised in that trichophytons or various fungi in a deep layer of the skin are allured by oxygen contained in the antifungal agent to or near the surface of the skin for easy and early sterilisation.

Another object of this invention is to provide an agent for activating the recovery of the skin after disease (infecticosa eczematoides or Engmaris disease) caused by trichophyton, eczema or various fungi or after burn injury which is characterised in that oxygen contained in the antifungal agent plays an important role in the recovery of the affected skin.

Another object of this invention is to provide an antifungal agent for the treatment of skin diseases such as athlete's foot, ringworm and tinea in safe and economic manner.

A further object of this invention is to provide a liquid agent (or lotion) for the treatment of a burn of the skin.

Still another object of this invention is to provide a liquid agent (or lotion) for the treatment of stiffness in the shoulders.

### 3. ESSENTIAL FEATURE OF THE INVENTION

The therapeutic agent of this invention comprises a hypochlorite such as an alkali metal hypochlorite, for example sodium hypochlorite; a sulfite, for example an alkali metal sulfite such as sodium sulfite; a nitrite, for example an alkali metal nitrite such as sodium nitrite; a chlorate, for example an alkali metal chlorate such as a mixture of sodium chlorate and potassium chlorate; hydrogen peroxide; ozone water; a nitrate, for example an alkali metal nitrate such as a mixture of sodium nitrate and potassium nitrate; a nonionic surface active agent such as a polyoxyethylene alkyl ether or phenyl ether; and water. It is characterised in that trichophytons or various fungi in a deep layer of the skin are allured by oxygen contained within the agent to or near the surface of the skin for easy sterilisation. It is also capable of activating the early recovery of the skin affected with dermatophytes, eczema or various fungi.

Ozone water as referred to above is obtained by dissolving ozone in water, for example to a concentration of 50 ppm by weight.

The agent according to the invention preferably contains 0.01-40 weight % of the hypochlorite; 0.01-30 weight % of the sulfite; 0.01-40 weight % of the nitrite; 0.01-40 weight % of the or each chlorate; 0.001-35 weight % of hydrogen peroxide; 0.01-40 weight % of ozone water; 0.01-40 weight % of the or each nitrate; 0.001-1 weight % of nonionic surface active agent; and 1-90% weight % of water. It may have a pH in the range 4-12.

### 4. EXAMPLES

### Example 1

A detergent solution of an antifungal agent for the treatment of skin diseases caused by trichophyton and eczema of this invention comprises a 100 weight % solution including 0.01-40 weight % of sodium hypochlorite, 0.01-30 weight % of sodium sulfite, 0.01-40 weight % of sodium nitrite, 0.01-40 weight % of sodium chlorate, 0.01-40 weight % of potassium chlorate, 0.001-35 weight % of hydrogen peroxide, 0.01-40 weight % of ozone water, 0.01-40 weight % of sodium nitrate, 0.01-40 weight % of potassium nitrate, 0.001-1 weight % of nonionic surface active agent and 1-90 weight % water.

### Example 2

A detergent solution of the antifungal agent for the treatment of skin diseases caused by trichiphyton and eczema described in Example 1 has pH value between 4-12.

### Example 3

The detergent solution of the antifungal agent was adjusted to have the pH value of 12. The adjusted solution was applied once a day to the flakey athlete's foot, oozey athlete's foot and cracked skin athlete's foot on the sole of a foot of a 48 year old man. After three days of its application, the trichophyton was completely sterilised, taking away not only the usual urtication of the athlete's foot, but also urtication felt when the body temperature of the patient rises.

At the same time, the skin affected with the athlete's foot recovered much earlier than the case treated with the conventional remedy for athlete's foot.

### Example 4

The aforementioned solution was applied once a day to eczema on the instep of a foot and on the back of a hand of a 55 year old woman, curing her of the diseases completely after about 3 days.

### Example 5

The aforementioned solution was applied once a day to dermatophyte and eczema of a toenail of a 55 year old man, having cured him of the diseases completely after about 10 days.

### Example 6

The aforementioned solution was applied once a day to dermatophyte on the palm of a man of 43 years, having cured him of the disease completely after about 3 days.

### Example 7

A cotton patch soaked with the aforementioned solution was applied at night on both shoulders of a 57 year old man who had been suffering from chronic stiffness in the shoulders, having facilitated the circulation of the blood to cure him of his chronic stiffness the next morning.

### Example 8

A 54 year old woman burnt her left hand near the thumb over a 3 square cm area while frying "Tempura" (or Japanese deep-fat fried food). She applied on the burn the aforementioned solution soaked in the cotton patch for one week to cure her of the burn completely.

## Claims

1. A therapeutic agent comprising 0.01-40 weight % of a hypochlorite; 0.01-30 weight % of a sulfite; 0.01-40% of a nitrite; 0.01-40 weight % of each of one or more chlorates; 0.001-35 weight % of hydrogen peroxide; 0.01-40 weight % of ozone water; 0.01-40 weight % of each of one or more nitrates; 0.001-1 weight % of nonionic surface active agent; and 1-90 weight % of water.

2. An agent as claimed in claim 1 which comprises 0.01-40 weight % of sodium hypochlorite, 0.01-30 weight % of sodium sulfite, 0.01-40 weight % of sodium nitrite, 0.01-40 weight % of sodium chlorate, 0.01-40 weight % of potassium chlorate, 0.001-35 weight % of hydrogen peroxide, 0.01-40 weight % of ozone water, 0.01-40 weight % of sodium nitrate, 0.01-40 weight % of potassium nitrate, 0.001-1 weight % of nonionic surface active agent and 1-90 weight % of water.

3. An agent as claimed in claim 1 or claim 2 having a pH value between 4-12.

4. An agent as claimed in any preceding claim for use in the treatment of skin disease such as athlete's foot, ringworm and tinea caused by dermatophytes, eczema, tinea or fungi.

5. An agent as claimed in any of claims 1 to 3 for the treatment of a burn on the skin of any portion of a human body.

6. An agent as claimed in any of claims 1 to 3 for the treatment of stiffness in the shoulders.

## Patentansprüche

1. Therapeutisches Mittel, umfassend 0,01 bis 40 Gew.-% eines Hypochlorits; 0,01 bis 30 Gew.-% eines Sulfits; 0,01 bis 40 % eines Nitrits; 0,01 bis 40 Gew.-% jeweils eines oder mehrerer Chlorate; 0,001 bis 35 Gew.-% Wasserstoffperoxid; 0,01 bis 40 Gew.-% Ozonwasser; 0,01 bis 40 Gew.-% jeweils eines oder mehrerer Nitrate; 0,001 bis 1 Gew.-% eines nichtionischen Tensids und 1 bis 90 Gew.-% Wasser.

2. Mittel nach Anspruch 1, umfassend 0,01 bis 40 Gew.-% Natriumhypochlorit, 0,01 bis 30 Gew.-% Natriumsulfit, 0,01 bis 40 Gew.-% Natriumnitrit, 0,01 bis 40 Gew.-% Natriumchlorat, 0,01 bis 40 Gew.-% Kaliumchlorat, 0,001 bis 35 Gew.-% Wasserstoffperoxid, 0,01 bis 40 Gew.-% Ozonwasser, 0,01 bis 40 Gew.-% Natriumnitrat, 0,01 bis 40 Gew.-% Kaliumnitrat, 0,001 bis 1 Gew.-% nichtionisches Tensid und 1 bis 90 Gew.-% Wasser.

3. Mittel nach Anspruch 1 oder Anspruch 2, mit einem pH-Wert zwischen 4-12.

4. Mittel nach einem vorangehenden Anspruch zur Verwendung bei der Behandlung von Hauterkrankungen, wie Fußpilz, Trichophytie, Tinea, hervorgerufen durch Dermatophyten, Ekzeme, Tinea oder Pilze.

5. Mittel nach einem der Ansprüche 1 bis 3 zur Behandlung von Hautverbrennungen eines Teils des menschlichen Körpers.

6. Mittel nach einem der Ansprüche 1 bis 3, zur Behandlung von Steifheit in den Schultern.

## Revendications

1. Un agent thérapeutique comprenant 0,01 à 40 % en poids d'un hypochlorite ; 0,01 à 30 % en poids d'un sulfite ; 0,01 à 40 % d'un nitrite ; 0,01 à 40 % en poids de chacun d'un ou plusieurs chlorates ; 0,001 à 35 % en poids de peroxyde d'hydrogène ; 0,01 à 40 % en poids d'eau ozonisée ; 0,01 à 40 % en poids de chacun d'un ou plusieurs nitrates ; 0,001 à 1 % en poids d'agent tensio-actif non ionique ; et 1 à 90 % en poids d'eau.

2. Un agent tel que revendiqué dans la revendication 1, qui comprend 0,01 à 40 % en poids d'hypochlorite de sodium, 0,01 à 30 % en poids de sulfite de sodium, 0,01 à 40 % en poids de nitrite de sodium, 0,01 à 40 % en poids de chlorate de sodium, 0,01 à 40 % en poids de chlorate de potassium, 0,001 à 35 % en poids de peroxyde d'hydrogène, 0,01 à 40 % en poids d'eau ozonisée, 0,01 à 40 % en poids de nitrate de sodium, 0,01 à 40 % en poids de nitrate de potassium, 0,001 à 1 % en poids d'agent tensio-actif non ionique et 1 à 90 % en poids d'eau.

3. Un agent tel que revendiqué dans la revendication 1 ou la revendication 2, ayant un pH compris entre 4 et 12.

4. Un agent tel que revendiqué dans l'une quelconque des revendications précédentes, pour son utilisation dans le traitement d'une maladie de la peau telle que le pied d'athlète, une dermatophytose et une teigne causés par des dermatophytes, un eczéma, une teigne ou des champignons.

5. Un agent tel que revendiqué dans l'une quelconque des revendications 1 à 3, pour le traitement d'une brûlure sur la peau de n'importe quelle partie du corps humain.

6. Un agent tel que revendiqué dans l'une quelconque des revendications 1 à 3, pour le traitement d'une raideur des épaules.
